# EUROPEAN PATENT SPECIFICATION

(11) **EP 2 440 178 B1**
(45) Date of publication and mention of the grant of the patent: **01.06.2016**
(21) Application number: 10735083.7
(22) Date of filing: 10.06.2010
(51) Int. Cl.: A61K 9/02

(54) **GLYCEROL-FREE OSMOTIC LAXATIVE SUPPOSITORY**
GLYCERINFREIES OSMOTISCHES LAXATIVES ZÄPFCHEN
SUPPOSITOIRE LAXATIF OSMOTIQUE SANS GLYCÉROL

(30) Priority: 10.06.2009 HU 0900353
(43) Date of publication of application: 18.04.2012
(73) Proprietor: Egis Gyógyszergyár Zrt., 1106 Budapest (HU)
(72) Inventor: MIKULÁSIK, Endre, H-8924 Alsónemesapáti (HU); SZAKÁLY, Péter, H-9900 Körmend (HU)
(74) Representative: Stolmár & Partner Patentanwälte PartG mbB
(86) International application number: PCT/HU2010/000066
(87) International publication number: WO 2010/143004

(56) References cited:
- GB-A- 917 456
- US-A1- 2004 265 344

## Description

### Field of the invention

The present invention relates to a laxative suppository which acts by an osmotic effect and contains polyethylene glycols (PEGs) instead of the generally used glycerol. The present invention also relates to a process for the preparation of the said suppositories.

### Technical background of the invention

Laxatives the effects of which being based on the stimulation of the rectum or the osmotic activity have been known since the ancient time. Several documents describe that introducing tallow or various oleaginous substances into the rectum enhances the defecation. Soapy enemas, with various additives are described in pharmaceutical-medicinal documents form the end of Middle Ages, their use being based on the irritative and lubricating effect of fatty soaps. However application of enemas was uncomfortable for the patients; disgusting and infectious for the operating personal and the used devices (e.g. clyster syringe) often injuring the mucous membrane of the rectum. Glycerol containing suppositories based on the same effect have been known since the middle of the 18^{th} century and the said products are more comfortable than enemas. Glycerol containing suppositories are manufactured by adding solid alkali to melted animal fats and glycerol, in an amount, wherein the melted mass will be able to congeal and can be poured. Nowadays glycerol suppositories are made by using stearic acid or palmitic acid, these suppositories are translucent, attractive and can be stored in appropriate packaging at low temperature for a long time. This receipt and preparation process is used widespreadly all over the world.

These types of suppositories start to liquefy after insertion into the rectum and osmotically absorb a large amount of water from the wall of the rectum while they become diluted. Due to the wetting effect of the soap the liquid penetrates into the dry faeces, soften it within about 20 minutes and due to the lubrication effect promotes the defecation.
The drug safety requirements and the demands of the patients are getting more and more serious. Glycerol, according to the present knowledge cannot be regarded as harmless. Glycerol above a concentration of 20 wt% irritates the mucous membrane and skin similarly to alcohol, and sometimes causes inflammation (in the glycerol suppositories the concentration of the glycerol is 80 wt%). The systemic effect of glycerol cannot be disregarded, because being absorbed it decreases the cerebral perfusion pressure, it can disadvantageously influence the condition of patients suffering from several diseases (e.g. epilepsy), especially on frequent use. The alkali used has an alkaline pH value and contains a certain amount of free alkali depending from the neutralization, which is particularly disadvantageous on regular use. A further reason for consideration is that the glycerol suppositories are used by babies and seriously ill elderly people, who are sensitive to side effects to a greater extent.

Thus there is a significant need for an osmotic laxative suppository, which is devoid of said side effects.

### Summary of invention

The object of the present invention is the provision of a Glycerol-free laxative suppository containing an osmotic component, a stabilizer and a wetting agent, wherein the stabilizer and the osmotic component are selected from PEG 200, 300, 400, 600, 1000, 1500, 2000, 3000 and 6000,wherein the glycerol-free laxative suppository contains 30 to 60 wt% of PEG 400, which suppository does not contain any further active ingredient.

### Detailed description of the invention

It has been surprisingly found that on using certain polyethylene glycols (PEGs) an osmotic laxative suppository which retains all the useful properties of glycerol suppositories can be prepared. Said suppositories are glycerol-free, storable at room temperature without liquefying and do not contain irritable ingredients.

Polyethylene glycols are well known and generally used polyethers. PEGs are manufactured by oligomerization or polymerization of ethylene oxide. PEGs are liquids or solids according to their molecular weight. The applicability and physical properties of the PEGs with different molecular weight can be varied by their chain-length, while their chemical properties are mostly identical. The use of PEGs is widespread due to their low toxicity. They are ingredients of food and cosmetic products, etc.

PEGs are universally used in the pharmaceutical industry, too. When coupled with proteins they can extend the release of proteins form blood, thus increasing the therapeutic effectiveness and decreasing toxicity. The combination of PEGs with electrolytes is used to clean the bowel before bowel surgery or colonoscopy. The use of PEGs as oral laxatives is also known. Several oral laxatives contain PEG 3350 (e.g. MiraLAX^{®} - Schering Plough).

It is known that PEGs are universally used as excipients of rectal, vaginal and other suppositories, such as BETADINE^{®} suppository (EGIS Gyógyszergyár Nyrt.), which contains as active principle Polyvidon-Jod and PEG as only auxiliary agent. All of these suppositories exert their therapeutic effect due to the active pharmaceutical ingredient, which is always present beside the PEGs. Stient et al. describe polyethylene based bisacodil containing laxative suppositories, wherein bisacodil is the laxative active ingredient. (Stient S.A., Luttrel W., Binard J.E. Spinal Cord (1998) 63, 777-781.).
From US 2004/265344 A1 there have been known laxative suppositories containing as active principle aloe and as auxiliary agents PEG's. In GB 917 456 A it has been described a suppository composition of low surface-activity consisting essentially of polyethylene glycol which is solid at normal temperatures, a salt of carbonic acid and an alkali metal acid phosphate or a salt of lactic acid, citric acid or tartaric acid.

It has been surprisingly found that polyethylene glycol, which is a non-toxic, well known and universally used ingredient, is able to exhibit the laxative effect per se without adding any active ingredient. Thus as a result of the present invention all the side effects of the active ingredient containing suppositories can be eliminated.

According to the present invention there is provided an osmotic component, stabilizer and wetting agent containing laxative suppository, wherein the osmotic component and the stabilizer is PEG 200 and a polyethylene glycol with higher molecular weight. The pharmaceutical composition of the present invention contains PEGs with 200/300/400/600/1000/1500/2000/3000/6000 molecular weight, preferably PEG 400/1500/6000.

According to the present invention there is provided a suppository, containing polyethylene glycol 400 instead of glycerol which does not irritate the mucous membrane, does not decrease the cerebral perfusion pressure and is not absorbed on the mucous membrane of the colon. At the same time PEG 400 - which can be safely used - has greater osmotic activity, and better hygroscopic properties than glycerol. The liquid and strongly hygroscopic PEG 400 is stabilized by addition polymers having higher molecular weight (PEG 1500 and PEG 6000), in order to make capable of forming suppositories, and the penetration thereof into the dry faeces is enhanced by adding wetting agent (Polysorbate [TWEEN^{®}] 60). All the three components are hygroscopic thus enhancing the osmotic activity. The pH of the suppository mass of the invention is neutral in contrast to glycerol suppositories.

The suppository of the present invention contains PEG 200/300/400/600/1000/1500/2000/3000/6000 as osmotic component and Polysorbate [TWEEN^{®}] 20/40/60/65/80/85 as wetting agent. The suppository of the present invention particularly preferably contains 30-60 wt% of PEG 400; 4,5-30 wt% of PEG 1500; 4,5-50 wt% of PEG 6000 and 0,1-5 wt% of Polysorbate [TWEEN^{®}] 60.
According a still more advantageous aspect the suppository of the present invention contains:

| | |
|---|---|
| PEG 6000 | 40 wt% |
| PEG 1500 | 19,5 wt% |
| PEG 400 | 40 wt% |
| Polysorbate [TWEEN^{®}] 60 | 0,5 wt% |

The suppository of the present invention is white, homogenous, has a shining surface and melting point of 40 to 42 °C, therefore it is solid at room temperature and is readily soluble in water and body fluids. A thin film of liquid PEG covers the surface of the suppository, which facilitates the insertion of the suppository into the rectum; even in case of inflamed and dry rectum there is no need to wetting.

The PEGs start to liquefy after insertion into the rectum and osmotically absorb a large amount of water from the wall of the rectum while they get diluted themselves. Due to Polysorbate [TWEEN^{®}] 60 the liquid penetrates into the dry and hard faeces and softens it. During defecation the ingredients of the composition act as lubricant thus assisting fast and painless defecation.

The posology of the suppository according to the present invention is identical to that of glycerol suppositories; according to need 1 to 2 suppositories are to be deeply inserted into the rectum and about 20 minutes are needed to display the effect while the buttocks should be pressed together. The defecation starts rapidly after relaxation of the buttocks. No irritation or inflammation occurs and the suppository has no effect on the cerebral circulation. The formulation is completely harmless to health; therefore it should not be worried if children swallow this.

The favourable properties of the present suppository of the present invention were demonstrated by the following experiments:
Model experiments were performed to demonstrate the features which were concluded from the physical-chemical properties of the suppository mass. Glycerol containing suppository of Biogal Pharmaceutical Plc was used as reference composition, which consists of stearic acid, palmitic acid and glycerol.

Exactly weighed 2 g on average suppositories were made from two suppository masses in a manual suppository mould, thus the differences between the two types of suppositories do not influence the results of the measurements. Each suppository was placed into a 10x6 cm (thickness 8 mm) cellftate plastic bag, which was pre-soaked in the experimental medium for 1 hour.

An isotonic NaCl solution tempered to 37 °C was used as test medium. The suppository containing bags were exactly weighed, closed and dipped 8 cm deeply into the tempered test solution. 6 suppositories from each type were tested and weighed after 10, 15 and 20 minutes, respectively, the weight of the absorbed water was compared to the exactly measured weight of the original suppository and expressed in % related to the equivalent suppository. Table 1 shows the results of the experiments.

| **Sample** | **Absorbed water (wt % on average)** | | |
|---|---|---|---|
| | After 10 minutes | After 15 minutes | After 20 minutes |
| Glycerol cont. suppository (Biogal) | 86,8 % | 98,2% | 102,6% |
| Suppository of the present invention | 98,8% | 108,4 % | 114,0% |

It has been surprisingly found that the *in vitro* osmotic activity of the suppository of the present invention is higher than that of the glycerol containing suppository.

Further details of the present invention are to be found in the following Examples.

### Examples:

### 1. Example

The composition of the suppository is as follows (related to one suppository, weight 2000 g):

| Compound | Amount |
|---|---|
| PEG 6000 | 800 mg |
| PEG 1500 | 390 mg |
| PEG 400 | 800 mg |
| Polysorbate [TWEEN^{®}] 60 | 10 mg |

### 2. Example

### Preparation of the suppository (~1 kg/500 suppositories)

The PEG 400 is heated to 50 °C and PEG 6000 and PEG 1500 are dissolved in the melted PEG 400. The Polysorbate [TWEEN^{®}] 60 is dissolved in the water-clean hot mixture.

The suppository mass is poured into an Erweka mould-type pre-covered PE cased PVC mould cavity and after congealing the mould is heat-sealed.

## Claims

1. Glycerol-free laxative suppository containing an osmotic component, a stabilizer and a wetting agent, wherein the stabilizer and the osmotic component are selected from PEG 200, 300, 400, 600, 1000, 1500, 2000, 3000 and 6000,wherein the glycerol-free laxative suppository contains 30 to 60 wt% of PEG 400.

2. Glycerol-free laxative suppository according to claim 1 containing 4,5 to 80 wt % stabilizer and 0,1 to 5 wt % wetting agent.

3. Glycerol-free laxative suppository according to claim 1, wherein the osmotic component is PEG 400 and the stabilizers are addition polymers having higher molecular weight (PEG 1500 and PEG 6000).

4. Glycerol-free laxative suppository according to claim 1 to 3, wherein the wetting agent is selected from polysorbate 20, 40, 60, 65, 80 and 85.

5. Glycerol-free laxative suppository according to claim 4, wherein the wetting agent is polysorbate 60.

6. Glycerol-free laxative suppository according to any one of the preceding claims, which comprises polysorbate 60, PEG 1500 and PEG 6000.

7. Glycerol-free laxative suppository according to any one of the preceding claims, which contains 40 wt % of PEG 400, 40 wt % of PEG 6000, 19,5 wt % of PEG 1500 and 0,5 wt % of polysorbate 60.

8. Process for the preparation of a suppository according to claims 1 to 7, which comprises dissolving the wetting agent in the melt of the stabilizer and the osmotic component, and stirring the melt and pouring the mass thus obtained into suppository mould.

## Patentansprüche

1. Glycerol-freies Abführzäpfchen enthaltend eine osmotische Komponente, einen Stabilisator und ein Feuchthaltemittel, wobei der Stabilisator und die osmotische Komponente ausgewählt sind aus PEG 200, 300, 400, 600, 1000, 1500, 2000, 3000, 6000, wobei das Glycerol-freie Abführzäpfchen 30-60 wt % von PEG 400 enthält.

2. Glycerol-freies Abführzäpfchen gemäß Anspruch 1 enthaltend 4,5-80 wt % Stabilisator und 0,1-5 wt % Feuchthaltemittel.

3. Glycerol-freies Abführzäpfchen gemäß Anspruch 1, wobei die osmotische Komponente PEG 400 ist und die Stabilisatoren zusätzliche Polymere sind, die ein höheres Molekulargewicht haben (PEG 1500 und PEG 6000).

4. Glycerol-freies Abführzäpfchen gemäß Anspruch 1 bis 3, wobei das Feuchthaltemittel ausgewählt ist aus Polysorbat 20, 40, 60, 65, 80 und 85.

5. Glycerol-freies Abführzäpfchen gemäß Anspruch 4, wobei das Feuchthaltemittel Polysorbat 60 ist.

6. Glycerol-freies Abführzäpfchen gemäß einem der vorangegangenen Ansprüche, welches Polysorbat 60, PEG 1500 und PEG 6000 umfasst.

7. Glycerol-freies Abführzäpfchen gemäß einem der vorangegangenen Ansprüche, welches 40 wt % PEG 400, 40 wt % PEG 6000, 19,5 wt % PEG 1500 und 0,5 wt % Polysorbat 60 enthält.

8. Verfahren zur Herstellung eines Zäpfchens gemäß Anspruch 1 bis 7, welches Auflösen des Feuchthaltemittels in der Schmelze des Stabilisators und der osmotischen Komponente und Rühren der Schmelze und Schütten der so erhaltenen Masse in eine Zäpfchenform umfasst.

## Revendications

1. Suppositoire laxatif exempt de glycérol contenant un composant osmotique, un stabilisant et un agent mouillant, dans lequel le stabilisant et le composant osmotique sont sélectionnés parmi PEG 200, 300, 400, 600, 1000, 1500, 2000, 3000 et 6000, dans lequel le suppositoire laxatif exempt de glycérol contient 30 à 60 % en poids de PEG 400.

2. Suppositoire laxatif exempt de glycérol selon la revendication 1 contenant 4,5 à 80 % en poids de stabilisant et 0,1 à 5 % en poids d'agent mouillant.

3. Suppositoire laxatif exempt de glycérol selon la revendication 1, dans lequel le composant osmotique est PEG 400 et les stabilisants sont des polymères d'addition ayant un poids moléculaire supérieur (PEG 1500 et PEG 6000).

4. Suppositoire laxatif exempt de glycérol selon les revendications 1 à 3, dans lequel l'agent mouillant est sélectionné parmi le polysorbate 20, 40, 60, 65, 80 et 85.

5. Suppositoire laxatif exempt de glycérol selon la revendication 4, dans lequel l'agent mouillant est le polysorbate 60.

6. Suppositoire laxatif exempt de glycérol selon l'une quelconque des revendications précédentes, qui comprend du polysorbate 60, PEG 1500 et PEG 6000.

7. Suppositoire laxatif exempt de glycérol selon l'une quelconque des revendications précédentes, qui contient 40 % en poids de PEG 400, 40 % en poids de PEG 6000, 19,5 % en poids de PEG 1500 et 0,5 % en poids de polysorbate 60.

8. Procédé pour la préparation d'un suppositoire selon les revendications 1 à 7, qui comprend dissoudre l'agent mouillant dans le bain du stabilisant et du composant osmotique, et agiter le bain et verser la masse ainsi obtenue dans le moule de suppositoire.
